(19) 

**Europäisches Patentamt**
**European Patent Office**
**Office européen des brevets**

(11) **EP 4 406 931 A1**

(12) **EUROPEAN PATENT APPLICATION**
published in accordance with Art. 153(4) EPC

(43) Date of publication:
**31.07.2024 Bulletin 2024/31**

(21) Application number: **22872707.9**

(22) Date of filing: **07.09.2022**

(51) International Patent Classification (IPC):
*C07C 4/00* (2006.01)     *C07C 11/00* (2006.01)
*C08J 11/12* (2006.01)     *C10G 1/10* (2006.01)

(52) Cooperative Patent Classification (CPC):
**C07C 4/00; C07C 11/00; C08J 11/12; C10G 1/10;**
**Y02W 30/62**

(86) International application number:
**PCT/JP2022/033505**

(87) International publication number:
**WO 2023/047951 (30.03.2023 Gazette 2023/13)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **24.09.2021 JP 2021155936**

(60) Divisional application:
**24181979.6**

(71) Applicants:
• **Muroran Institute of Technology**
  **Muroran-shi**
  **Hokkaido 050-8585 (JP)**
• **Sumitomo Chemical Company, Limited**
  **Tokyo 103-6020 (JP)**

(72) Inventors:
• **KANDA, Yasuharu**
  **Muroran-shi, Hokkaido 050-8585 (JP)**
• **UEMICHI, Yoshio**
  **Muroran-shi, Hokkaido 050-8585 (JP)**
• **ISHIHARA, Nozomi**
  **Ichihara-shi, Chiba 299-0195 (JP)**
• **NAKASUJI, Takehiro**
  **Niihama-shi, Ehime 792-8521 (JP)**

(74) Representative: **Plasseraud IP**
  **66, rue de la Chaussée d'Antin**
  **75440 Paris Cedex 09 (FR)**

(54) **OLEFIN PRODUCTION METHOD**

(57)     To enable highly efficient production of an olefin with a low environmental impact. The production includes a thermal cracking step of thermal cracking plastic to obtain a thermally cracked gas (G1) and a catalytic cracking step of cracking the thermally cracked gas (G1) in the presence of a catalyst to obtain a catalytically cracked gas (G2). When a ratio of an olefin to paraffin in the thermally cracked gas (G1) is indicated as R1 and a ratio of an olefin to paraffin in the catalytically cracked gas (G2) is indicated as R2, the following formula is satisfied: R2/R1 > 1.

FIG. 1

PREPROCESSING — S12

THERMAL CRACKING — S13

CATALYTIC CRACKING — S14

PURIFICATION — S15

EP 4 406 931 A1

**Description**

Technical Field

The present invention relates to an olefin production method.

Background Art

In order to realize a carbon-cycle society, attention has been focused on technologies for chemical recycling of waste plastic, which has traditionally been thermally recycled. The mainstream of such technologies is a method which, as disclosed in, for example, Patent 1 or non-Patent 1, hydrogenates a cracked product obtained by cracking waste plastic and introduces the hydrogenated cracked product into a naphtha cracker to obtain an olefin.

Citation List

[Patent Literature]

[Patent Literature 1]
Japanese Patent No. 6378368

[Non-patent Literature]

[Non-patent Literature 1]
ChemCycling @ BASF SE "Plastic Waste Pyrolysis-Challenges along the process chain", Freiberger Pyrolysetag, 18. 9. 2019, Martin Gall, Sebastian Schulze BASF SE (https://tu-freiberg.de/sites/default/files/media/institut-fuer-energieverfahrenstechnik-143/EVT/Schulungen/pyrolyse19/03._gall_schulze_basf_pyr olysetag.pdf)

Summary of Invention

Technical Problem

However, the cracked product obtained by the thermal cracking of the waste plastic is a mixture containing an olefin, paraffin, an aromatic compound, and the like. As such, a hydrogenation step is required in order to increase a yield of the olefin which is a product obtained by using the naphtha cracker with the cracked product as a raw material. As a result, hydrogen, which has a large environmental impact, is required.
It is an object of an aspect of the present invention to produce an olefin highly efficiently without using hydrogen.

Solution to Problem

In order to attain the object, a method in accordance with an aspect of the present invention is a method for producing an olefin from plastic containing a polyolefin, the method including: a thermal cracking step of thermally cracking the plastic to obtain a thermally cracked gas; and a catalytic cracking step of cracking the thermally cracked gas in the presence of a catalyst to obtain a catalytically cracked gas, wherein $R2/R1 \geq 1$ where $R1$ is a weight ratio of an olefin having 2 to 30 carbon atoms to paraffin having 1 to 30 carbon atoms in the thermally cracked gas, and $R2$ is a weight ratio of an olefin having 2 to 30 carbon atoms to paraffin having 1 to 30 carbon atoms in the catalytically cracked gas.
Further, a method in accordance with an aspect of the present invention is a method for producing an olefin from plastic containing a polyolefin, the method including: a cracking step of thermally cracking the plastic to obtain a cracked gas, wherein a temperature $T4$ of the cracked gas at a gas outlet of a cracking device in which the cracking step is carried out is not lower than a dew point of the cracked gas.
Further, a method in accordance with an aspect of the present invention is a method for producing an olefin from plastic containing a polyolefin, the method including: a cracking step of thermally cracking the plastic to obtain a cracked gas, wherein $T5-T6 \leq 200$ (°C) where $T5$ (°C) is a cracking temperature set in the cracking step, and $T6$ (°C) is a temperature of the cracked gas at a gas outlet of a cracking device in which the cracking step is carried out.

Advantageous Effects of Invention

In accordance with an aspect of the present invention, it is possible to produce an olefin highly efficiently without using hydrogen in production of an olefin from plastic containing a polyolefin.

Brief Description of Drawings

Fig. 1 is a flowchart illustrating an example of an olefin production method in accordance with Embodiment 1 of the present invention.
Fig. 2 is a system diagram schematically illustrating a configuration of a production system in accordance with Embodiment 1 of the present invention.
Fig. 3 is a flowchart illustrating an example of an olefin production method in accordance with Embodiment 2 of the present invention.
Fig. 4 is a system diagram schematically illustrating a configuration of a production system in accordance with Embodiment 2 of the present invention.

Description of Embodiments

Embodiment 1

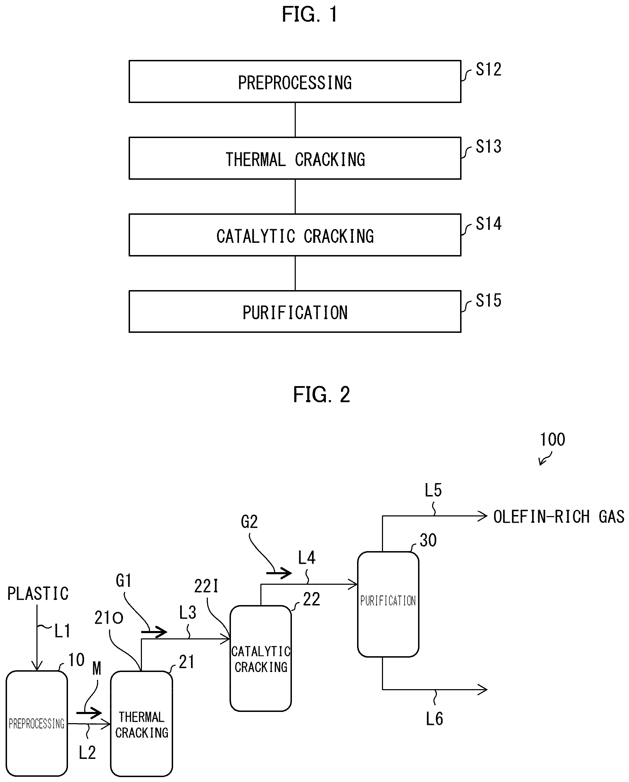

The following description will discuss in detail, with reference to drawings, an olefin production method in accordance with an embodiment of the present invention as well as a production system used in the olefin production method.
The olefin production method in accordance with the present embodiment is a method for producing, with use of plastic such as waste plastic as a raw material, a lower olefin that can be in the carbon cycle as a plastic raw material. Fig. 1 is a flowchart illustrating an example of the olefin production method in accordance with the present embodiment. As illustrated in Fig. 1, the olefin production method in accordance with the present embodiment includes a preprocessing step S12, a thermal cracking step S13, a catalytic cracking step S14, and a purification step S15. Each of these steps will be detailed later.
The description in the present embodiment will discuss, for example, an olefin production system (Fig. 2: production system 100) which makes it possible to implement the flowchart illustrated in Fig. 1 and a production flow indicated by the flowchart. However, the system described in the present specification and drawings merely serves as a typical example, and does not limit the scope of the present invention in any way. The same is true of the other embodiments below.

<Olefin production system (production system 100)>

First, the following description will discuss, with reference to Figs. 1 and 2, an example of a configuration of the production system 100. Fig. 2 is a system diagram schematically illustrating a configuration of the production system 100 in accordance with Embodiment 1.
The production system 100 in accordance with the present embodiment is a system which cracks plastic, in particular a polyolefin-based plastic, to obtain an olefin-rich gas that is rich in lower olefin gas. The plastic used as a raw material in the olefin production method in accordance with the present embodiment can be, for example, waste plastic. Further, the plastic used as a raw material preferably is a polyolefin-based plastic such as polyethylene, polypropylene, or the like, and a content ratio of the polyolefin-based plastic is preferably not less than 80% by mass.
As illustrated in Fig. 2, the production system 100 in accordance with the present embodiment is schematically configured to include a preprocessing system 10, a thermal cracking device 21, a catalytic cracking device 22, a purification device 30, and lines L1 to L6.
The preprocessing system 10 is supplied with plastic such as waste plastic from the line L1. A discharge opening of the preprocessing system 10 and a feed opening of the thermal cracking device 21 are connected to each other via the line L2. A material M to be supplied which has been preprocessed by the preprocessing system 10 is supplied to the thermal cracking device 21 through the line L2. A gas outlet 210 of the thermal cracking device 21 and a feed opening 22I of the catalytic cracking device 22 are connected to each other via the line L3. The material M supplied to the thermal cracking device 21 is thermally cracked and supplied to the catalytic cracking device 22 through the line L3 as a thermally cracked gas G1. The catalytic cracking device 22 and the purification device 30 are connected to each other via the line L4. The thermally cracked gas G1 supplied to the catalytic cracking device 22 is catalytically cracked and supplied to the purification device 30 as a catalytically cracked gas G2. The catalytically cracked gas G2 is purified by the purification device 30, and an olefin-rich gas that is rich in a lower olefin and an oil-containing liquid are discharged through the line L5 and the line L6, respectively. The following will discuss the devices (system) in detail.

The preprocessing system 10 is a system which processes plastic such as waste plastic into the material M to be supplied that is suitable for being subjected to cracking. That is, the preprocessing system 10 is a system which implements the preprocessing step S12. The preprocessing system 10 can include a plurality of devices which carry out respective different processes. For example, the preprocessing system 10 can include one or more devices selected from the group consisting of: a selection device; a crushing device; a cleaning device; a drying device; a melting device; and a dechlorinaion device. The selection device is a device which selects a polyolefin-based plastic from, for example, a raw material such as waste plastic. As the selection device, it is possible to use, for example, at least one selected from the group consisting of an optical selection device, a densimetric separation device, and the like. The crushing device is, for example, a device that crushes selected plastic. The cleaning device is, for example, a device that cleans crushed plastic. The drying device is, for example, a device that dries cleaned plastic. The melting device is a device that heats plastic into a liquid state. The dechlorinaion device is a device that removes chlorine contained in plastic.

The thermal cracking device 21 is a device which cracks and vaporizes a substance by heating. That is, the thermal cracking device 21 is a device capable of carrying out the thermal cracking step S13 in accordance with the present embodiment. The thermal cracking device 21 can be, for example, an extruder, a stirred tank, a rotary kiln, a fluidized bed, or the like. The fluidized bed can be an internally-circulating fluidized bed or an externally-circulating fluidized bed. Further, it is possible to use a plurality of devices among these devices listed as examples, and the plurality of reactors can be connected in parallel or in series. As a heat source necessary in the thermal cracking step S13, it is possible to use heat obtained by burning any one or more selected from the group consisting of: a thermally cracked residue generated in the thermal cracking device 21; a hydrocarbon-containing liquid and/or hydrocarbon-containing lower paraffin gas obtained in the purification step S15; or a hydrocarbon fuel such as natural gas or kerosene. Alternatively, it is possible to use, as the heat source, heat obtained by electric heating or microwave irradiation. Alternatively, it is possible to make a combined use of at least two selected from the group consisting of: heat obtained from electric heating; heat obtained from heating by microwave irradiation; and heat obtained from the above-described burning. The method of heating can be direct heating or indirect heating. Examples of the direct heating include a method in which a microwave-absorbing substance (susceptor) is retained in the device and microwave energy is supplied to the waste plastic via the substance. Examples of the indirect heating include: a method in which heat of an electric heater or heat obtained by burning a hydrocarbon fuel is supplied via a heat transfer surface of the device; and a method in which water vapor or an inert gas such as nitrogen gas or $CO_2$ gas is heated to a high temperature by a heat source in advance and then introduced into the device. Further, it is also possible to use a method in which a solid mainly containing iron, iron oxide, alumina, silica, or the like is preheated to a high temperature by a heat source and then introduced into the device. The preheating of gas or solid particles by a heat source can be carried out by using a device similar to a part of an inside of the thermal cracking device 21 or to the thermal cracking device and combining the device with the thermal cracking device 21 to cause gas or a solid in these devices to circulate.

The catalytic cracking device 22 is a device which cracks a substance by bringing a thermally cracked gas and a catalyst into contact with each other. That is, the catalytic cracking device 22 is a device which implements the catalytic cracking step S14. The catalytic cracking device 22 can be, for example, a fixed bed, a moving bed, or a fluidized bed. Further, a plurality of reactors selected from the group consisting of these reactors listed as examples can be used, and the plurality of reactors can be connected in parallel or in series. As a heat source necessary in the catalytic cracking step S14, it is possible to use heat obtained by burning any one or more selected from the group consisting of: coke generated in the catalytic cracking device 22 and attached to a surface of the catalyst; a hydrocarbon-containing liquid and/or hydrocarbon-containing lower paraffin gas obtained in the purification step S15; and a hydrocarbon fuel such as natural gas or kerosene. Alternatively, it is possible to use, as a heat source, heat obtained by electric heating or microwave irradiation. Alternatively, it is possible to make a combined use of at least two selected from the group consisting of: heat obtained from electric heating; heat obtained from heating by microwave irradiation; and heat obtained from the above-described burning. The method of heating can be direct heating or indirect heating. Examples of the direct heating include a method in which a microwave-absorbing substance (susceptor) is retained in the device and microwave energy is supplied to the waste plastic via the substance. Examples of the indirect heating include: a method in which heat of an electric heater or heat obtained by burning a hydrocarbon fuel is supplied via a heat transfer surface of the device; and a method in which water vapor or an inert gas such as nitrogen gas or $CO_2$ gas is heated to a high temperature by a heat source in advance and then introduced into the device. Further, it is also possible to use a method in which a solid mainly containing iron, iron oxide, alumina, silica, or the like is preheated to a high temperature by a heat source and then introduced into the device. The solid particles can be the above catalyst. The preheating of gas or solid particles by a heat source can be carried out by using a device similar to a part of an inside of the thermal cracking device 21 or to the thermal cracking device and combining the device with the thermal cracking device 21 to cause gas or a solid in these devices to circulate.

The purification device 30 is a device capable of separating a mixture supplied to the purification device 30 by a known gas-liquid separation operation, a known distillation operation, or the like. That is, the purification device 30 is a device capable of carrying out the purification step S15 in accordance with the present embodiment. The purification device 30 can be, for example, a gas-liquid separation device or a distillation device. These devices can be used in combination, and a plurality of these devices can be connected to each other.

<Olefin production method>

The olefin production method in accordance with Embodiment 1 is carried out, for example, in accordance with the flowchart illustrated in Fig. 1. The flowchart illustrated in Fig. 1 is an example and the present embodiment is not limited to this example. The following will describe steps in the olefin production method in accordance with Embodiment 1 in details.

(Raw material)

Prior to description of each step, the following will discuss a raw material used in an embodiment of the present invention. A raw material supplied to the preprocessing system 10 can be plastic. The plastic can be waste plastic included in municipal waste or the like. The plastic preferably mainly contains a polyolefin such as polyethylene or polypropylene, but can contain another plastic. Examples of the another plastic can include: a chlorinated plastic (such as chlorinated polyethylene); polyvinyl chloride (PVC); polyvinylidene chloride (PVDC); a non-chlorinated plastic (e.g. polyethylene, polypropylene, polyethylene terephthalate (PET), polybutylene terephthalate, polystyrene, nylon 66, or the like); and a mixture thereof. Further, the waste plastic can include an unused mixed plastic or a used mixed plastic.

(Preprocessing step S12)

The preprocessing step S12 is a step of preprocessing the plastic to obtain the material M to be supplied and subjected to the thermal cracking step S13. The plastic which has been subjected to the preprocessing step S12 can be supplied to the thermal cracking step S13 as the material M to be supplied that mainly contains a polyolefin-based plastic. The preprocessing step preferably achieves a content ratio of a polyolefin-based plastic of not less than 80% by mass.

(Thermal cracking step S13)

The thermal cracking step S13 is a step of cracking the supplied material M by heating to obtain the thermally cracked gas G1. More specifically, the thermal cracking step S13 is a step of producing the thermally cracked gas G1 by heating the olefin-based plastic contained in the supplied material M to thereby crack the olefin-based plastic into hydrocarbon with a low molecular weight having approximately 1 to 30 carbon atoms. A thermal cracking temperature T1 (°C) in the thermal cracking step S13 can be set in accordance with a composition of the supplied material M. The thermal cracking temperature T1 is preferably a high temperature at which plastic is cracked at a high cracking rate, but an excessively high temperature causes carbonization. As such, for example, the thermal cracking temperature T1 is preferably 400°C to 800°C, and more preferably 400°C to 550°C. A pressure in the thermal cracking step S13 is preferably low because the cracking reaction is a reaction in which the number of moles increases. The pressure in the thermal cracking step S13 is, for example, -80 kPa to 1000 kPa, preferably -10 kPa to 300 kPa, and more preferably 0 kPa to 100 kPa.

In the thermal cracking step S13, water vapor or an inert gas such as nitrogen gas or $CO_2$ gas can coexist, and these gasses can be used as a fluidizing gas for the fluidized bed.

In thermal cracking, reflux allows a high boiling point component to be condensed and recracked so that the number of carbon atoms can be adjusted, to some extent, so as to make it easy to handle the high boiling point component as a fuel or a raw material for a naphtha cracker. In conventional technologies, therefore, examples of actively employing reflux are known.

The inventors of the present invention found, as a result of diligent research, that reflux in thermal cracking reduces R1. R1 means a weight ratio of an olefin to paraffin in a thermally cracked product. In other words, the inventors of the present invention found that reducing flux in thermal cracking makes it possible to increase R1.

In conventional technologies, a raw material rich in paraffin is preferable in order to increase an olefin yield in a naphtha cracker. It is therefore preferable to reduce R1 by reflux. However, in the olefin production method in accordance with an embodiment of the present invention, an increase in R1 causes an increase in weight ratio R2 of an olefin to paraffin in a catalytically cracked product obtained by catalytically cracking a thermally cracked product, and thus causes an increase in yield of a lower olefin. In view of the above, the inventors of the present invention found that reducing reflux is important in order to increase an olefin yield in the production method in accordance with an embodiment of the present invention.

According to an embodiment of the present invention, an increase in R1 makes it possible to obtain gas that is rich in a lower olefin. This makes it possible to produce an olefin highly efficiently without using hydrogen.

In the thermal cracking step S13, a reflux ratio can be, for example, not more than 0.1. By reducing reflux in the thermal cracking step S13 and setting the reflux ratio to not more than 0.1, it is possible to increase R1. Note here that R1 is a weight ratio of an olefin having 2 to 30 carbon atoms to paraffin having 1 to 30 carbon atoms contained in the thermally cracked gas G1. The reflux ratio refers to a ratio of (i) to (ii): (i) a weight ratio of a condensed thermally cracked product reintroduced into the thermal cracking device 21; and (ii) a weight ratio of a thermally cracked product discharged from the thermal cracking device 21. The thermally cracked product refers to a cracked product derived from plastic and does not include an inert gas and the like.

In order to reduce reflux in the thermal cracking step S13, for example, a temperature T2 (°C) of the thermally cracked gas G1 at the gas outlet 210 of the thermal cracking device 21 can be maintained so as to satisfy the following formula (1).

$$(1): T1\text{-}T2 \leq 200 \ (°C)$$

Alternatively, in order to reduce reflux in the thermal cracking step S13, the temperature T2 of the thermally cracked gas G1 at the gas outlet 210 of the thermal cracking device 21 can be maintained at not lower than a dew point of the thermally cracked gas G1. The dew point of the thermally cracked gas G1 refers to a temperature at which condensation starts at a thermal cracking pressure. In the thermal cracking step S13, the thermally cracked gas G1 vaporizes from a liquid phase due to vapor-liquid equilibrium, and it is therefore possible to assume that the dew point = a cracking temperature. The dew point varies in accordance with a composition ratio in the thermally cracked gas G1 and a pressure. In a case where an olefin-based plastic is used as a main raw material, "not lower than the dew point of the thermally cracked gas G1" can mean a temperature range of not lower than 200°C, preferably not lower 300°C, and more preferably not lower 350°C.

The above configuration makes it possible to reduce reflux in the thermal cracking step S13 and to increase R1. In order to reduce reflux in the thermal cracking step S13, the temperature, a velocity of the thermally cracked gas G1, and the like can be actively controlled between the gas outlet 210 of the thermal cracking device 21 and the feed opening 22I of the catalytic cracking device 22. For example, one or more of the following (i) to (vi) can be employed as a configuration for reducing reflux.

(i) The thermal cracking device 21 and/or a gas outlet pipe (line L3) are/is heated or kept warm.

(ii) A linear velocity of the thermally cracked gas G1 discharged from the thermal cracking device 21 is increased. For example, at least part of the line L3 preferably has a pipe diameter that achieves a linear velocity of not less than 1.0 m/s, and more preferably has a pipe diameter that achieves a linear velocity of not less than 10 m/s.

(iii) An inclination of the line L3 is designed such that even if condensation occurs in the line L3, no condensate is reintroduced into the thermal cracking device 21.

(iv) The temperature of the thermally cracked gas G1 is maintained at not lower than the dew point of the thermally cracked gas G1 in the line L3 between the thermal cracking device 21 and the catalytic cracking device 22.

(v) Time during which the thermally cracked gas G1 stays in the line L3 is reduced (preferably not longer than 30 seconds, and more preferably not longer than 10 seconds).

By thus reducing reflux in the thermal cracking step S13, it is possible to achieve R1 of not less than 1.0. R1 being not less than 1.0 makes it possible to obtain gas that is rich in an olefin.

(Catalytic cracking step S14)

The catalytic cracking step S14 is a step capable of producing the catalytically cracked gas G2 by cracking the thermally cracked gas G1 in the presence of a catalyst into hydrocarbon with a low molecular weight having approximately 1 to 20 carbon atoms. A cracking temperature T3 in the catalytic cracking step S14 can be set in accordance with a composition of the thermally cracked gas G1. The cracking temperature T3 can be, for example, 400°C to 800°C. The catalyst used in the catalytic cracking step S14 can be, but are not limited to, a silicate catalyst, preferably a zeolite catalyst, and more preferably an MFI-type zeolite catalyst. The silicate catalyst can typically contain a silicon atom, an aluminum atom, an oxygen atom, and a hydrogen atom. Further, the silicate catalyst can contain atoms such as a sodium atom, a titanium atom, a chromium atom, a manganese atom, an iron atom, a cobalt atom, a nickel atom, a copper atom, a ruthenium atom, a rhodium atom, a palladium atom, a silver atom, an iridium atom, a platinum atom, a boron atom, a nitrogen atom, a magnesium atom, a phosphorus atom, a zinc atom, and a gallium atom.

In the catalytic cracking step S14, water vapor or an inert gas such as nitrogen gas or $CO_2$ gas can coexist,

and these gasses can be used as a fluidizing gas for the fluidized bed.

The inventors of the present invention found that in a case where the catalytic cracking step S14 is carried out after the thermal cracking step S13, the following formula is satisfied: R2/R1 ≥ 1.

Note that R2 is a weight ratio of an olefin having 2 to 30 carbon atoms to paraffin having 1 to 30 carbon atoms contained in the catalytically cracked gas G2.

(Purification step S15)

The purification step S15 is a step of separating and purifying the catalytically cracked gas G2. More specifically, the purification step S15 is a step capable of separating the catalytically cracked gas G2 into (i) a gas containing at least one hydrocarbon having a small number of carbon atoms (e.g. $C_{1-5}$) and (ii) a liquid containing at least one hydrocarbon having a large number of carbon atoms (e.g. not less than $C_6$). Further, the gas can be an olefin-rich gas containing not less than 90% by mass of a lower olefin. The lower olefin can contain at least one selected from the group consisting of: ethylene; propylene; and butene.

Since the olefin-rich gas that is rich in a lower olefin can be obtained, it is possible to produce an olefin highly efficiently without using hydrogen.

Note that at least part of the hydrocarbon-containing liquid obtained in the purification step S15 can be supplied to the thermal cracking device 21 or the catalytic cracking device 22. This makes it possible to further increase the olefin yield.

Alternatively, the hydrocarbon-containing liquid and/or the hydrocarbon-containing lower paraffin gas obtained in the purification step S15 can be burned to be used as a heat source in any step among the preprocessing step S12 to the purification step S15. This makes it possible to reduce an environmental impact of the entire olefin production system.

Embodiment 1 is a method for producing an olefin from plastic containing a polyolefin, the method including: the thermal cracking step S13 of preprocessing and thermal cracking the plastic to obtain the thermally cracked gas G1; and the catalytic cracking step S14 of cracking the thermally cracked gas G1 in the presence of a catalyst to obtain the catalytically cracked gas G2. When a ratio of an olefin having 2 to 30 carbon atoms to paraffin having 1 to 30 carbon atoms in the thermally cracked gas G1 is indicated as R1 and a ratio of an olefin having 2 to 30 carbon atoms to paraffin having 1 to 30 carbon atoms in the catalytically cracked gas G2 is indicated as R2, the following formula is satisfied: R2/R1 ≥ 1.

With such a configuration, it is possible to produce an olefin highly efficiently without using hydrogen. This makes it possible to contribute to achieving the Sustainable Development Goals (SDGs).

Embodiment 2

The following description will discuss another embodiment of the present invention. For convenience of explanation, the same reference signs will be given to members each having the same function as a member described in the above embodiment, and descriptions on such a member will be omitted. The same applies to the other embodiments below.

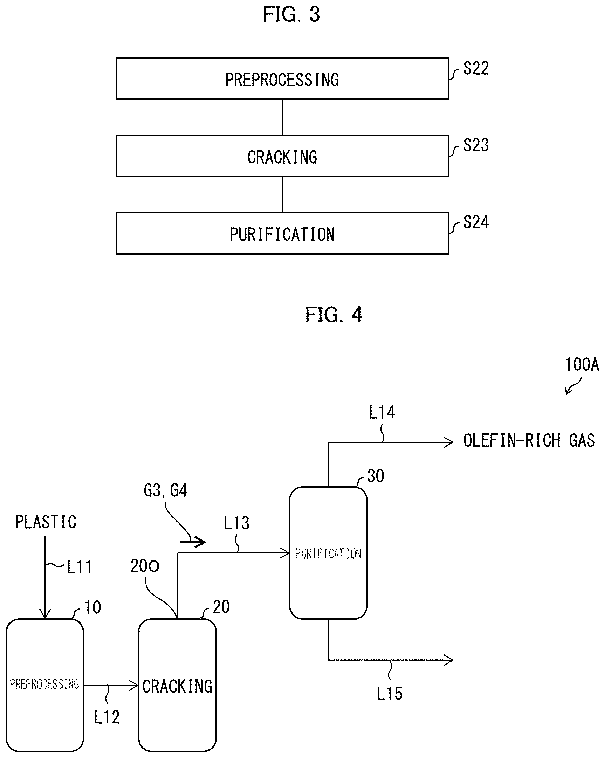

Fig. 3 is a flowchart illustrating an example of an olefin production method in accordance with Embodiment 2. As illustrated in Fig. 3, the olefin production method in accordance with Embodiment 2 differs from Embodiment 1 in that a cracking step S23 is not divided, unlike the thermal cracking step S13 and the catalytic cracking step S14 in accordance with Embodiment 1.

Fig. 4 is a system diagram schematically illustrating a configuration of the olefin production system in accordance with Embodiment 2 (production system 100A). The production system 100A is schematically configured to include a preprocessing system 10, a cracking device 20, a purification device 30, and lines L11 to L15.

The preprocessing system 10 is supplied with plastic such as waste plastic from the line L11. A discharge opening of the preprocessing system 10 and a feed opening of the cracking device 20 are connected to each other via the line L12. A material M to be supplied which has been preprocessed by the preprocessing system 10 is supplied to the cracking device 20 through the line L12. A gas outlet 20O of the catalytic cracking device 20 and the purification device 30 are connected to each other via the line L13. The material M supplied to the cracking device 20 is cracked and supplied to the purification device 30 as a cracked gas G3. The cracked gas G3 is purified by the purification device 30, and an olefin-rich gas that is rich in lower paraffin and an oil-containing liquid are discharged through the line L14 and the line L15, respectively.

The cracking device 20 is a device which cracks and vaporizes a substance by heating. The cracking device 20 can be the thermal cracking device 21 or the catalytic cracking device 22 in accordance with Embodiment 1.

The olefin production method in accordance with Embodiment 2 is carried out, for example, in accordance with the flowchart illustrated in Fig. 3. In the preprocessing step S22, processes respectively identical to those in the preprocessing step S12 in accordance with Embodiment 1 can be carried out. Description of the same step as in Embodiment 1 is omitted, and the cracking step S23 and the purification step S24 will be explained below.

(Cracking step S23)

The cracking step S23 is a step of obtaining the cracked gas G3 by cracking the supplied material M (i) by heating alone or (ii) by heating and contacting with a catalyst.

More specifically, the cracking step S23 is a step of producing the cracked gas G3 by cracking, (i) by heating alone or (ii) by heating and contacting with a catalyst, an olefin-based plastic contained in the supplied material M into hydrocarbon with a low molecular weight having approximately 1 to 20 carbon atoms.

The cracking device 20 in which the cracking step S23 is carried out can be the thermal cracking device 21 or the catalytic cracking device 22 described in Embodiment 1. Alternatively, the cracking device 20 can be a cracking tank in which the thermal cracking step S13 and the catalytic cracking step S14 in accordance with Embodiment 1 can be carried out in the same reaction tank. In this case, catalytic cracking can occur in the liquid phase. Further, it is possible to use the heat source described in Embodiment 1.

In the cracking step S23, water vapor or an inert gas such as nitrogen gas or $CO_2$ gas can coexist, and these gasses can be used as a fluidizing gas for the fluidized bed.

In the cracking step S23, a temperature T4 (°C) of the cracked gas G3 at the gas outlet 20O of the cracking device 20 is not lower than a dew point of the cracked gas G3. In order to maintain T4 at not lower than the dew point of the cracked gas G3, for example, the cracking device 20 and/or a gas outlet pipe (line L13) can be heated or kept warm. Further, in the cracking step S23, a reflux ratio can be set to not more than 0.1. By setting the reflux ratio to not more than 0.1, it is possible to increase a weight ratio R3 of an olefin having 2 to 30 carbon atoms to paraffin having 1 to 30 carbon atoms in the cracked gas G3.

(Purification step S24)

The purification step S24 is a step of separating and purifying the cracked gas G3. More specifically, the purification step S24 is a step capable of separating the cracked gas G3 into (i) a gas containing at least one hydrocarbon having a small number of carbon atoms (e.g. $C_{1-5}$) and (ii) a liquid containing at least one hydrocarbon having a large number of carbon atoms (e.g. not less than $C_6$). Further, the gas can be an olefin-rich gas containing not less than 90% by mass of a lower olefin. The lower olefin can contain at least one selected from the group consisting of: ethylene; propylene; and butene.

Since the olefin-rich gas that is rich in a lower olefin can be obtained, it is possible to produce an olefin highly efficiently without using hydrogen.

Embodiment 2 is a method for producing an olefin from plastic containing a polyolefin, the method including the cracking step S23 of thermal cracking the plastic to obtain the cracked gas G3, and the temperature T4 of the cracked gas G3 at the gas outlet 20O of the cracking device 20 in which the cracking step S23 is carried out is not lower than the dew point of the cracked gas G3.

This configuration makes it possible to reduce reflux in the cracking step S23 and to increase R3 in the cracked gas G3 obtained by cracking the plastic. This makes it possible to produce an olefin highly efficiently without using hydrogen.

Embodiment 3

The olefin production method in accordance with Embodiment 3 can be carried out, for example, in accordance with the flowchart illustrated in Fig. 3, as in Embodiment 2.

When a cracking temperature in a cracking step S23 is indicated as T5 (°C), a temperature T6 of a cracked gas G4 at a gas outlet of a cracking device 20 in the cracking step S23 in accordance with Embodiment 3 is in a temperature range satisfying T5-T6 $\leq$ 200 (°C).

This configuration makes it possible to reduce reflux in the cracking step S23 and to increase a weight ratio O/P (R4) of an olefin having 2 to 30 carbon atoms to paraffin having 1 to 30 carbon atoms in the cracked gas G4 obtained by cracking plastic. This makes it possible to produce an olefin highly efficiently without using hydrogen.

Aspects of the present invention can also be expressed as follows:

(1) An olefin production method in accordance with Aspect 1 of the present invention is a method for producing an olefin from plastic containing a polyolefin, the method including: a thermal cracking step of thermally cracking the plastic to obtain a thermally cracked gas; and a catalytic cracking step of cracking the thermally cracked gas in the presence of a catalyst to obtain a catalytically cracked gas, wherein R2/R1 $\geq$ 1 where R1 is a weight ratio of an olefin having 2 to 30 carbon atoms to paraffin having 1 to 30 carbon atoms in the thermally cracked gas, and R2 is a weight ratio of an olefin having 2 to 30 carbon atoms to paraffin having 1 to 30 carbon atoms in the catalytically cracked gas.

(2) In Aspect 2 of the present invention, the olefin production method in accordance with Aspect 1 is configured such that T1-T2 ≤ 200 (°C) where T1 (°C) is a thermal cracking temperature set in the thermal cracking step, and T2 (°C) is a temperature of the thermally cracked gas at a gas outlet of a thermal cracking device in which the thermal cracking step is carried out.

(3) In Aspect 3 of the present invention, the olefin production method in accordance with Aspect 1 or 2 is configured such that a temperature T2 of the thermally cracked gas at a gas outlet of a thermal cracking device in which the thermal cracking step is carried out is not lower than a dew point of the thermally cracked gas.

(4) In Aspect 4 of the present invention, the olefin production method in accordance with any one of Aspects 1 through 3 is configured such that a temperature of the thermally cracked gas is maintained at not lower than a dew point of the thermally cracked gas between the thermal cracking step and the catalytic cracking step.

(5) In Aspect 5 of the present invention, the olefin production method in accordance with any one of Aspects 1 through 4 is configured such that a reflux ratio in the thermal cracking step is not more than 0.1.

(6) In Aspect 6 of the present invention, the olefin production method in accordance with any one of Aspects 1 through 5 is configured such that R1 is not less than 1.0.

(7) In Aspect 7 of the present invention, the olefin production method in accordance with any one of Aspects 1 through 6 is configured such that the method further includes a purification step of separating the catalytically cracked gas to obtain an olefin-rich gas containing not less than 90% by mass of a lower olefin, the lower olefin containing at least one selected from the group consisting of: ethylene; propylene; and butene.

(8) An olefin production method in accordance with Aspect 8 of the present invention is a method for producing an olefin from plastic containing a polyolefin, the method including: a cracking step of thermally cracking the plastic to obtain a cracked gas, wherein a temperature T4 of the cracked gas at a gas outlet of a cracking device in which the cracking step is carried out is not lower than a dew point of the cracked gas.

(9) An olefin production method in accordance with Aspect 9 of the present invention is a method for producing an olefin from plastic containing a polyolefin, the method including: a cracking step of thermally cracking the plastic to obtain a cracked gas, wherein T5-T6 ≤ 200 (°C) where T5 (°C) is a cracking temperature set in the cracking step, and T6 (°C) is a temperature of the cracked gas at a gas outlet of a cracking device in which the cracking step is carried out.

(10) In Aspect 10 of the present invention, the olefin production method in accordance with Aspect 8 or 9 is configured such that a reflux ratio in the cracking step is not more than 0.1.

(11) In Aspect 11 of the present invention, the olefin production method in accordance with any one of Aspects 8 through 10 is configured such that the method further includes a purification step of separating the cracked gas to obtain an olefin-rich gas containing not less than 90% by mass of a lower olefin, the lower olefin containing at least one selected from the group consisting of: ethylene; propylene; and butene.

The present invention is not limited to the embodiments, but can be altered by a skilled person in the art within the scope of the claims. The present invention also encompasses, in its technical scope, any embodiment derived by combining technical means disclosed in differing embodiments.

(Verification test)

The following will describe a result of an experiment on a relationship between a temperature at a gas outlet of a thermal cracking device (also, a cracking device) and an O/P ratio.

Table 1 summarizes experimental conditions and experimental results of Experimental Examples 1 to 5.

[Table 1]

| Experimental Example No. | No. 1 | No. 2 | No. 3 | No. 4 | No. 5 |
|---|---|---|---|---|---|
| Thermal cracking temperature T1 (also, T5) [°C] | 435 | 435 | 435 | 460 | 460 |
| Temperature T2 at outlet of thermal cracking device (also, T6) [°C] | 300 | 240 | 198 | 310 | 185 |
| T1-T2 (also, T5-T6) [°C] | 135 | 195 | 237 | 150 | 275 |
| R1 (also, R3, R4) [-] | 1.2 | 1 | 0.7 | 1.3 | 1.2 |
| Yield of olefin having 2 to 5 carbon atoms in thermally cracked product [%] | 4.9 | 2.1 | 2.0 | 4.0 | 3.8 |

(continued)

| Experimental Example No. | No. 1 | No. 2 | No. 3 | No. 4 | No. 5 |
|---|---|---|---|---|---|
| R2 [-] | - | - | 2.5 | 3.8 | 2.8 |
| R2/R1 [-] | - | - | 3.5 | 2.7 | 2.7 |
| Yield of olefin having 2 to 5 carbon atoms in catalytically cracked product [%] | - | - | 42 | 54 | 45 |

Devices used were the same among all of Experimental Examples Nos. 1 to 5. A reaction tube made of glass was used as the thermal cracking device. The reaction tube was filled with 1.0 g of polyethylene (manufactured by Sumitomo Chemical Co., Ltd., Sumikasen (registered trademark) G201G), which is a polyolefin-based plastic. A cold trap was connected downstream of the reaction tube, and a 2-L gas bag was connected to a downstream side of the cold trap. A temperature of a resin in the reaction tube (= a thermal cracking temperature T1 (also, a cracking temperature T5)) was 435°C in Experimental Examples Nos. 1 to 3 and 460°C in Experimental Examples Nos. 4 and 5. Thermal cracking of the polyethylene was carried out while nitrogen gas was caused to flow from an upstream side of the reaction tube at a rate of 10 ml/min. In so doing, an amount of an insulating material at an outlet part of the reaction tube, which outlet part was directed to the cold trap, was caused to change such that a temperature T2 at the outlet of the reaction tube (also, T6) was adjusted to 300°C, 240°C, 198°C, 310°C, and 185°C in Experimental Examples Nos. 1 to 5, respectively.

One hour after a start of the heating of the polyethylene, thermally cracked products in liquid and wax states obtained were collected in the cold trap, and a thermally cracked product in a gaseous state obtained was collected in the gas bag. The collected thermally cracked products in liquid, wax, and gaseous states were each analyzed by gas chromatography to measure an olefin content and a paraffin content. From measured results of these contents, R1 (also, R3, R4) and a yield of an olefin having 2 to 5 carbon atoms were calculated. The calculation results are shown in Table 1.

Regarding Experimental Examples Nos. 1 to 3, at a thermal cracking temperature of 435°C, a yield of an olefin having 2 to 5 carbon atoms in a thermally cracked product in each of Nos. 1 and 2, in which $T5-T6 \leq 200°C$, was higher than that in No. 3, in which $T5-T6 > 200°C$. Regarding Experimental Examples Nos. 4 and 5, a yield of an olefin having 2 to 5 carbon atoms in a thermally cracked product in accordance with No. 4, in which $T1-T2 \leq 200°C$, was higher than that in accordance with No. 5, in which $T1-T2 > 200°C$.

Subsequently, in Experimental Examples Nos. 3 to 5, the products in liquid and wax states obtained by the thermal cracking of the polyethylene were introduced into a reaction tube made of glass, and catalytic cracking was carried out. Since a weight of the product in a gaseous state obtained by the thermal cracking of the polyethylene was not more than 10% of a weight of the raw material polyethylene, catalytic cracking was carried out on the assumption that only the products in liquid and wax states were a thermally cracked product. The catalytic cracking was carried out such that the reaction tube made of glass was heated at a temperature equal to the thermal cracking temperature to cause the thermally cracked product to be evaporated, and the thermally cracked product thus evaporated was brought into contact with an MFI-type zeolite catalyst (Si/Al = 400). R2 and a yield of an olefin having 2 to 5 carbon atoms were calculated in a similar manner to R1 (also, R3, R4).

It was verified that a yield of a lower olefin having 2 to 5 carbon atoms in each of the catalytically cracked products of Nos. 3 to 5, in each of which R2/R1 (also, R3, R4) $\geq 1$ was satisfied due to catalytic cracking, was higher than a yield of a lower olefin having 2 to 5 carbon atoms which was obtained by thermal cracking alone.

In the catalytically cracked product of No. 4, in which $T1-T2 \leq 200°C$, a lower olefin having 2 to 5 carbon atoms was obtained in a yield higher than that in each of the catalytically cracked products of Nos. 3 and 5, in each of which $T1-T2 > 200°C$.

In each of the catalytically cracked products in accordance with Nos. 4 and 5, in each of which $R1 \geq 1.0$, a lower olefin having 2 to 5 carbon atoms was obtained in a yield higher than that in the catalytically cracked product in accordance with No. 3, in which $R1 < 1.0$.

Reference Signs List

    100, 100A: production system
    10: pretreatment system
    20: cracking device
    21: thermal cracking device

20O, 21O: gas outlet
22: catalytic cracking device
G1: thermally cracked gas
G2: catalytically cracked gas
G3, G4: cracked gas

**Claims**

1.  A method for producing an olefin from plastic containing a polyolefin, the method comprising:

    a thermal cracking step of thermally cracking the plastic to obtain a thermally cracked gas; and
    a catalytic cracking step of cracking the thermally cracked gas in the presence of a catalyst to obtain a catalytically cracked gas,
    wherein $R2/R1 \geq 1$ where R1 is a weight ratio of an olefin having 2 to 30 carbon atoms to paraffin having 1 to 30 carbon atoms in the thermally cracked gas, and R2 is a weight ratio of an olefin having 2 to 30 carbon atoms to paraffin having 1 to 30 carbon atoms in the catalytically cracked gas.

2.  The method as set forth in claim 1, wherein $T1-T2 \leq 200$ (°C) where T1 (°C) is a thermal cracking temperature set in the thermal cracking step, and T2 (°C) is a temperature of the thermally cracked gas at a gas outlet of a thermal cracking device in which the thermal cracking step is carried out.

3.  The method as set forth in claim 1, wherein a temperature T2 of the thermally cracked gas at a gas outlet of a thermal cracking device in which the thermal cracking step is carried out is not lower than a dew point of the thermally cracked gas.

4.  The method as set forth in claim 1, wherein a temperature of the thermally cracked gas is maintained at not lower than a dew point of the thermally cracked gas between the thermal cracking step and the catalytic cracking step.

5.  The method as set forth in claim 1, wherein a reflux ratio in the thermal cracking step is not more than 0.1.

6.  The method as set forth in claim 1, wherein R1 is not less than 1.0.

7.  The method as set forth in claim 1, further comprising:

    a purification step of separating the catalytically cracked gas to obtain an olefin-rich gas containing not less than 90% by mass of a lower olefin,
    the lower olefin containing at least one selected from the group consisting of: ethylene; propylene; and butene.

8.  A method for producing an olefin from plastic containing a polyolefin, the method comprising:

    a cracking step of thermally cracking the plastic to obtain a cracked gas,
    wherein a temperature T4 of the cracked gas at a gas outlet of a cracking device in which the cracking step is carried out is not lower than a dew point of the cracked gas.

9.  The method as set forth in claim 8, wherein a reflux ratio in the cracking step is not more than 0.1.

10.  A method for producing an olefin from plastic containing a polyolefin, the method comprising:

    a cracking step of thermally cracking the plastic to obtain a cracked gas,
    wherein $T5-T6 \leq 200$ (°C) where T5 (°C) is a cracking temperature set in the cracking step, and T6 (°C) is a temperature of the cracked gas at a gas outlet of a cracking device in which the cracking step is carried out.

11.  The method as set forth in claim 10, wherein a reflux ratio in the cracking step is not more than 0.1.

12.  The method as set forth in any one of claims 8 through 11, further comprising:

    a purification step of separating the cracked gas to obtain an olefin-rich gas containing not less than 90% by mass of a lower olefin,
the lower olefin containing at least one selected from the group consisting of: ethylene; propylene; and butene.

## FIG. 1

| | |
|---|---|
| PREPROCESSING | S12 |
| THERMAL CRACKING | S13 |
| CATALYTIC CRACKING | S14 |
| PURIFICATION | S15 |

## FIG. 2

# FIG. 3

| | |
|---|---|
| PREPROCESSING | S22 |
| CRACKING | S23 |
| PURIFICATION | S24 |

# FIG. 4

100A

L14 → OLEFIN-RICH GAS

PLASTIC

G3, G4

L11

200

L13

30

10

20

PURIFICATION

PREPROCESSING → CRACKING

L12

L15

<table>
<tr><td colspan="2">**INTERNATIONAL SEARCH REPORT**</td><td colspan="2">International application No.<br>**PCT/JP2022/033505**</td></tr>
</table>

**A. CLASSIFICATION OF SUBJECT MATTER**

*C07C 4/00*(2006.01)i; *C07C 11/00*(2006.01)i; *C08J 11/12*(2006.01)i; *C10G 1/10*(2006.01)i
FI: C08J11/12; C07C4/00; C07C11/00; C10G1/10

According to International Patent Classification (IPC) or to both national classification and IPC

**B. FIELDS SEARCHED**

Minimum documentation searched (classification system followed by classification symbols)

C07C4/00; C07C11/00; C08J11/12; C10G1/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | Document 2: 上道芳夫 他. ポリオレフィンの接触分解による低級オレフィン化. ファインケミカル. 2017, 46(12), pp. 44-51, https://doi.org/10.11523/sekiyu.2017f.0_163 (KAMIMICHI, Yoshio et al. Catalytic Degradation of Polyolefins into Lower Olefins. Fine Chemical)<br>    pp. 46-49 | 1-12 |
| X | 加賀慎之介 他. ポリエチレンの接触分解による低級オレフィン化. 石油学会年会・秋季大会講演要旨集, 2017, 第47回石油・石油化学討論会 (鳥取), 190, https://doi.org/10.11523/sekiyu.2017f.0_163, session ID 2D23 (KAGA, Shinnosuke et al. Catalytic degradation of polyethylene into lower olefins. Proceeding of Annual/Fall Meetings of the Japan Petroleum Institute 2017, 47th Petroleum-Petrochemical Symposium of JPI (Tottori))<br>    p. 190 | 1-12 |
| X | WO 2021/166854 A1 (SUMITOMO CHEMICAL COMPANY, LIMITED) 26 August 2021 (2021-08-26)<br>    examples | 1-12 |

☐ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **04 October 2022** | **18 October 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2022/033505**

| Box No. III | Observations where unity of invention is lacking (Continuation of item 3 of first sheet) |
|---|---|

This International Searching Authority found multiple inventions in this international application, as follows:

Document 1: 加賀慎之介 他, ポリエチレンの接触分解による低級オレフィン化, 石油学会年会・秋季大会講演要旨集, 2017, 第47回石油・石油化学討論会 (鳥取), 190, https://doi.org/10.11523/sekiyu.2017f.0_163, session ID 2D23, (KAGA, Shinnosuke et al. Catalytic degradation of polyethylene into lower olefins. Proceeding of Annual/Fall Meetings of the Japan Petroleum Institute 2017, 47th Petroleum-Petrochemical Symposium of JPI (Tottori))

The claims are classified into the following three inventions.
(Invention 1) Claims 1-7
Claims 1-7 are classified as invention 1 as a result of having the special technical feature in which, with regard to a pyrolysis gas, when the weight ratio of olefins having 2-30 carbon atoms relative to paraffins having 1-30 carbon atoms is noted as R1, and with regard to a catalytic decomposition gas, when the weight ratio of olefins having 2-30 carbon atoms relative to paraffins having 1-30 carbon atoms is noted as R2, $R2/R1 \geq 1$ is satisfied.
(Invention 2) Claims 8-9
Claims 8-9 share, with claim 1 classified as invention 1, the feature of a method for manufacturing olefins from plastic containing polyolefins, the method including a decomposition step for pyrolyzing the plastic and obtaining a decomposition gas. However, this feature does not make a contribution over the prior art in the light of the content disclosed in document 1, and thus this feature cannot be said to be a special technical feature. Furthermore, there is no other identical or corresponding special technical feature among these inventions.
In addition, claims 8-9 do not depend from claim 1. Moreover, claims 8-9 are not substantially identical to or similarly closely related to any of the claims classified as invention 1.
Therefore, claims 8-9 cannot be classified as invention 1.
Claims 8-9 are classified as invention 2 as a result of having the special technical feature in which a temperature T4 of a decomposition gas at a gas outlet of a decomposition device for carrying out a decomposition step is at least a dew point temperature of the decomposition gas.
(Invention 3) Claims 10-12
Claims 10-12 share, with claim 1 classified as invention 1 and claim 8 classified as invention 2, the feature of being a method for manufacturing olefins from plastic containing polyolefins, the method including a decomposition step for pyrolyzing the plastic and obtaining a decomposition gas. However, this feature does not make a contribution over the prior art in the light of the content disclosed in document 1, and thus this feature cannot be said to be a special technical feature. Furthermore, there is no other identical or corresponding special technical feature among these inventions.
Additionally, claims 10-12 do not depend from claim 1 or 8. Moreover, claims 10-12 are not substantially identical to or similarly closely related to any of the claims classified as invention 1 or 2.
Accordingly, claims 10-12 cannot be classified as either invention 1 or 2.
Claims 10-12 are classified as invention 3 as a result of having the special technical feature in which, when the decomposition temperature set in the decomposition step is noted as T5 (°C), and the temperature of the decomposition gas at the gas outlet of the decomposition device for carrying out the decomposition step is noted as T6 (°C), $T5-T6 \leq 200$ (°C) is satisfied.

Form PCT/ISA/210 (continuation of first sheet) (January 2015)

EP 4 406 931 A1

**INTERNATIONAL SEARCH REPORT**

International application No.

**PCT/JP2022/033505**

| Box No. III | Observations where unity of invention is lacking (Continuation of item 3 of first sheet) |

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

17

**INTERNATIONAL SEARCH REPORT**
Information on patent family members

International application No.

**PCT/JP2022/033505**

| Patent document cited in search report | Publication date (day/month/year) | Patent family member(s) | Publication date (day/month/year) |
|---|---|---|---|
| WO 2021/166854 A1 | 26 August 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6378368 B

**Non-patent literature cited in the description**

- **MARTIN GALL ; SEBASTIAN SCHULZE.** Plastic Waste Pyrolysis-Challenges along the process chain. *Freiberger Pyrolysetag,* 18 September 2019, https://tu-freiberg.de/sites/default/files/media/institut-fuer-energieverfahrenstechnik-143/EVT/Schulungen/pyrolyse19/03._gall_schulze_basf_pyr olysetag.pdf